(19) Europäisches Patentamt / European Patent Office / Office européen des brevets

(11) **EP 3 883 486 B1**

(12) **EUROPEAN PATENT SPECIFICATION**

(45) Date of publication and mention of the grant of the patent:
**25.10.2023   Bulletin 2023/43**

(21) Application number: **19805701.0**

(22) Date of filing: **22.11.2019**

(51) International Patent Classification (IPC):
**A61B 18/02** *(2006.01)*     **A61B 18/14** *(2006.01)*
**A61M 25/00** *(2006.01)*     **A61B 18/00** *(2006.01)*

(52) Cooperative Patent Classification (CPC):
**A61B 18/02; A61B 18/1492;** A61B 2018/00166;
A61B 2018/00351; A61B 2018/00357;
A61B 2018/00375; A61B 2018/00577;
A61B 2018/00875; A61B 2018/0212;
A61B 2018/0262; A61B 2018/1407;
A61B 2018/1435; A61B 2217/005

(86) International application number:
**PCT/EP2019/082289**

(87) International publication number:
**WO 2020/104679 (28.05.2020 Gazette 2020/22)**

(54) **ABLATION DEVICE WITH ADJUSTABLE ABLATION APPLICATOR SIZE AND ABLATION SYSTEM**

ABLATIONSVORRICHTUNG MIT EINSTELLBARER ABLATIONSAPPLIKATORGRÖSSE UND ABLATIONSSYSTEM

DISPOSITIF D'ABLATION À TAILLE D'APPLICATEUR D'ABLATION RÉGLABLE ET SYSTÈME D'ABLATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **22.11.2018   EP 18207897**

(43) Date of publication of application:
**29.09.2021   Bulletin 2021/39**

(73) Proprietor: **AFreeze GmbH**
**6020 Innsbruck (AT)**

(72) Inventors:
• **FISCHER, Gerald**
**6176 Völs (AT)**
• **NIKE NOWAK, Claudia**
**6068 Mils (AT)**
• **DENNY, William**
**6082 Patsch (AT)**

(74) Representative: **Dilg, Haeusler, Schindelmann Patentanwaltsgesellschaft mbH**
**Leonrodstraße 58**
**80636 München (DE)**

(56) References cited:
EP-A2- 2 252 228     WO-A1-2017/211915
WO-A2-2016/196066     US-A- 5 800 428
US-A1- 2002 111 618     US-A1- 2018 085 160
US-B2- 9 918 772

EP 3 883 486 B1

**Description**

The present invention relates to the field of medical devices. More specifically, the present invention relates to an ablation device. The present invention further relates to an ablation system.

Ablation is the controlled modification of tissue by application of heat or extreme cold for therapeutic application. Different types of energy, such as radio-frequency (RF) electric current, ultra-sound heating or laser, can be used to apply heat. Cryo-ablation uses extreme cold for modifying tissue by ice-formation within body-liquids in the extra- and intracellular space. Tissue of isotonic osmolality freezes at temperatures below -0.6°C (Prickett et al.: Cryobiology 2010). Cryo-ablation offers the possibility of evenly distributing refrigerant along a cryo-applicator of elongated shape. For example, for the treatment of supraventricular tachycardia, cryo-thermal energy triggers the replacement of electrically conducting myocardial tissue by non-conducting connective tissue. Elongated non-conducting lesions restore a normal heart rhythm in patients with atrial fibrillation or atrial flutter. For the treatment of atrial fibrillation, various types of loop shaped elongated catheters are described in the art (see e.g. US 2005/0010095 A1 and US 9 918 772). For treatment of atrial flutter, cryo-applicators of a curved elongated line shape are described in the art (see e.g. US 9 011 425). Various mechanisms are known for adjusting the shape of an elongated ablation catheter to the shape and size of the tissue to be treated (see e.g. US 6 071 274, EP 2 252 228 A2, and WO 2016/196066 A2).

Also for diagnostic purposes, elongated catheters are described in the art (see e.g.US 2017/0291008). Here, electrodes spaced along a line are used for testing the outcome of ablation therapy by measuring electric signals or by pacing tissue. Often such diagnostic measurements are not performed directly at the lesion and it may be beneficial to adjust the size of the diagnostic catheter for measuring or pacing in tissue adjacent to the lesion. In US 8 475 450, a loop-shaped catheter of adjustable size is described which can be used for both radio frequency ablation and diagnostic recordings. Here, a pull-wire mechanism is used for adjusting the size. It is a well-known drawback of radio frequency ablation that electrodes may over heat and potentially trigger thromboembolic events. Therefore, the electrodes are usually flushed with a saline solution for reducing heating to an acceptable value. This technique is called irrigation. Although irrigation provides cooling, this technique is clearly distinguished from cryo-ablation. While cryo-ablation aims for the creation of ice at sub-zero temperatures, irrigation prevents for overheating well above body temperature. As described in US 8 475 450, the flushing solution for irrigation is directly delivered into the body. That potentially causes a significant load of liquid infused into the body when creating elongated lesions. In contrast, for cryo-ablation the boiled refrigerant is not allowed to escape into the body. Instead, to maintain patient safety, it has to be drained from the catheter at a low pressure.

For maintaining sufficiently low pressures inside the boiling and draining lumina of a cryo-ablation catheter, a sufficiently large cross section must be provided with the boiling and draining lumina. Thus, the use of a pull wire for controlling shape and/or size of a therapeutically active distal segment of a cryo-ablation catheter provides the disadvantage that it will partially occupy cross sections in the shaft and, therefore, may increase the dimension of the catheter shaft to a possibly unacceptable large size. Furthermore, the refrigerant may have significant impact on the temperature of a pull wire which is guided in parallel with the refrigerant. Temperature may induce significant shortening or lengthening of the pull wire which in turn may lead to an undesired change of the shape or size of the distal treatment portion.

As an alternative to pull wires, torsional mechanisms for changing the size and/or shape of loop shaped catheter structures are described in the art. In US 8 475 450, a torsional mechanism is described for a diagnostic double loop catheter. In US 6 071 274, pull wires and torsional mechanisms are described for radio frequency ablation catheters with adjustable loop shape.

However, such mechanisms cannot be directly applied to cryo-ablation catheters. When transmitting a torque or a rotational force to a cryo-therapy segment of adjustable size/shape, proper sealing of the cryo-therapy segment has to be provided in order to prevent an undesired escape of refrigerant. Furthermore, the sealing structure must also be able to withstand pull forces applied for stretching the device to an essentially linear configuration during insertion or removal.

Thus, there may be a need for a way of adjusting shape and size of a cryo-applicator without sacrificing safety of operation.

This need may be met by the subject-matter set forth in appended independent claim 1.

According to an exemplary embodiment of the invention, an ablation device is provided. The ablation device comprises (a) an ablation applicator for applying ablation energy to an ablation region, the ablation applicator having a first applicator end portion, a second applicator end portion, and an elongate portion extending between the first and second applicator end portions, (b) a first catheter having a first distal end portion and a first proximate end portion, the first distal end portion being coupled to the first applicator end portion, and (c) a second catheter having a second distal end portion and a second proximate end portion, the second distal end portion being coupled to the second applicator end portion to supply an ablation medium to the ablation applicator, (d) wherein the ablation applicator is adapted to form an applicator shape having an applicator size in a plane perpendicular to a longitudinal axis of the first catheter, and (e) wherein the coupling between the first distal end portion and the first applicator end portion is adapted to transfer a rotational force applied to the first proximate portion to the ablation applicator and thereby cause the applicator size to change.

According to another exemplary embodiment, an ablation system is provided. The ablation system comprises (a) an ablation device having the above-mentioned features, and (b) an ablation medium source adapted to supply the ablation medium to the second catheter.

The term "ablation device" may particularly denote any apparatus which is adapted to ablate, deactivate, destroy or remove material, particularly tissue of a physiological object such as a human being or an animal, via the application of an ablation medium, such as extreme cold or heat, radio frequency (RF) electric current, a laser beam, etc.

The term "ablation applicator" may particularly denote a structural part for applying ablation energy or medium to the ablation region, i.e. to tissue to be ablated. The ablation applicator may in particular be an integral or dedicated part of an ablation catheter, such as a tip of the catheter, or a separate part connected with the ablation catheter.

The term "applicator shape" may particularly denote a 3-dimensional geometrical shape of the ablation applicator.

The term "applicator size in a plane perpendicular to a longitudinal axis of the first catheter" may particularly denote a size, such as a width or diameter, of a projection of the applicator shape onto the plane that is substantially perpendicular to the longitudinal axis of the first catheter. In other words, the term "applicator size" relates to the dimensions of the projection of the ablation applicator and not the dimension in the axial direction.

According to an exemplary embodiment of the invention, an ablation device comprises an ablation applicator for applying ablation energy to an ablation region (tissue to be ablated), a first catheter and a second catheter. The ablation applicator has a first applicator end portion, a second applicator end portion, and an elongate portion extending between the first and second applicator end portions. In other words, the ablation applicator is an essentially elongate member with respective end portions. The first catheter has a first distal end portion and a first proximate end portion, and the second catheter has a second distal end portion and a second proximate end portion. The (first and second) distal end portions are the end portions of the respective (first and second) catheters closest to the ablation applicator and thus to the ablation region during operation. The (first and second) proximate end portions are the end portions of the respective (first and second) catheters farthest from the ablation applicator, typically located outside an insertion site of a patient. The first distal end portion (of the first catheter) is (mechanically) coupled to the first applicator end portion. The second distal end portion (of the second catheter) is coupled to the second applicator end portion to supply an ablation medium, such as a cryogenic fluid, to the ablation applicator. The ablation applicator is adapted to form (or take on) an applicator shape. The applicator shape has an applicator size in a plane perpendicular to a longitudinal axis of the first catheter, i.e. a size in a radial direction relative to the longitudinal axis. The (mechanical) coupling between the first distal end portion (of the first catheter) and the first applicator end portion is adapted to transfer a rotational force applied to the first proximate end portion (of the first catheter) to the ablation applicator and thereby cause the applicator size to change. In other words, when the first proximate end portion of the first catheter is rotated (about the longitudinal axis of the first catheter), a corresponding rotation takes place at the first distal end portion of the first catheter and a corresponding force is transferred to the first applicator end portion. This causes the applicator size to change. It is noted that the rotation at the first distal end portion may be less than the rotation of the first proximate portion due to torsion of the first catheter.

Next, further exemplary embodiments of the ablation device will be explained and discussed. However, these embodiments also apply to the ablation system and to the method.

The ablation applicator may be pre-shaped to a predetermined applicator shape. In other words, the predetermined applicator shape may be a default applicator shape, i.e. a shape which the ablation applicator will try to take on whenever possible.

The ablation applicator may comprise a shape memory structure defining the predetermined applicator shape. The shape memory structure has been pretreated (e.g. by heating and/or cooling in combination with shaping) to have an inherent shape corresponding to the predetermined applicator shape.

In some embodiments, the shape memory structure may comprise a wire made from a shape memory material, such as nitinol. The wire extends within a tube forming the ablation applicator. It should be noted that the tube may apply some force to the shape memory structure causing it to deviate slightly from the predetermined shape. To compensate for this and to minimize stress on the shape memory structure, a default amount of rotation may be applied to the first proximate end portion of the first catheter to counteract the force applied by the applicator tubing. Thereby, it is assured that the predetermined applicator shape corresponds to the inherent shape of the shape memory structure. This shape may also be referred to as a natural shape.

The predetermined applicator shape may be selected from the group consisting of a loop shape, a helical shape, and an arc shape. The projection of the loop shape and the helical shape onto a plane perpendicular to the longitudinal axis of the first catheter may be essentially circular or essentially elliptic, while the projection of the arc shape may be an essentially straight line extending radially away from the longitudinal axis. In some embodiments, the predetermined applicator shape may not be (completely) rotationally symmetric relative to the longitudinal catheter axis.

The first catheter may be a positioning catheter and the second catheter may be an ablation catheter. In other words, the first catheter may serve to position the ablation applicator relative to the ablation region while the second catheter may serve to deliver ablation energy to the applicator. The positioning catheter and the ablation catheter may be arranged in parallel within a sheath. Furthermore, the positioning catheter and the ablation catheter may be axially displaceable relative to one another. The ablation catheter may also be referred to as catheter shaft or just shaft.

The ablation catheter may comprise a supply lumen adapted to supply a cryogenic ablation medium to the ablation applicator and a draining lumen adapted to drain the cryogenic ablation medium from the ablation applicator.

The supply lumen may be formed by a supply tube extending within a larger tube which may define the draining lumen.

A cross-sectional area of the draining lumen may be larger than a cross-sectional area of the supply lumen. In particular, the cross-sectional area of the draining lumen may be equal to three times the cross-sectional area of the supply lumen or more, more particularly equal to six times the cross-sectional area of the supply lumen or more.

The ablation applicator may comprise a first lumen in communication with the supply lumen and a second lumen in communication with the draining lumen, the second lumen comprising a plurality of outlet openings adapted to provide a flow of cryogenic medium to the second lumen with a pressure drop causing the cryogenic medium to boil within the second lumen.

In other words, the cryogenic fluid flows from the supply lumen of the ablation catheter into the first lumen of the ablation applicator, through the outlet openings into the second lumen of the ablation applicator and back through the draining lumen of the ablation catheter. Due to the pressure drop, the second lumen acts both as a boiling chamber and as a draining lumen.

The ablation device may further comprise a stopping member arranged within the first applicator end portion to close the second lumen. In other words, the stopping member constitutes the distal end of the second lumen (boiling chamber).

The ablation device may further comprise a guiding wire extending within the first catheter and adapted to facilitate positioning of the ablation applicator within a cavity, such as an ostium of a vessel.

The ablation device may further comprise a plurality of electrodes arranged on a surface of the ablation applicator. The electrodes may in particular be evenly distributed across the surface. In some embodiments, each electrode is connected to an electrical wire extending towards the proximate end of the first or second catheter, such that signals can be supplied to and/or read from each single electrode.

The plurality of electrodes may in particular be adapted to at least one of: (i) sensing an electrical signal within the ablation region, (ii) supplying an electrical signal to the ablation region, (iii) supplying electrical ablation energy to the ablation region, and (iv) obtaining an impedance mapping of the ablation region.

Thus, the electrodes may be useful in a number of ways for determining electrical properties of the tissue (pre and post ablation), for electrically stimulating (pacing) the tissue, and for (additional) ablation of tissue.

The plurality of electrodes may also be useful in conjunction with fluoroscopy, i.e. medical imaging during operation. The fluoroscopy may involve any standard imaging method, such as x-ray (single-plane, bi-plane or rotational (similar to computed tomography)), electro-anatomical mapping (EAM), ultrasound or even magnetic resonance imaging (MRI). EAM involves the use of magnetic fields or impedance measurements for assessing a 3D-location (e.g. x, y, z) of a catheter electrode. Signals and locations are measured for a plurality of sites (typical some tens to more than hundred). Data is displayed on a 3D map (colored 3D-geometry with color code indicating for example maximal amplitude at each side).

More specifically, impedance measurements can be used for coupling the catheter with electro-anatomical navigation or mapping. For example the geometry of the applicator may be shown superimposed on a three-dimensional geometry. The approximate location of an electrode may be displayed within the map. This information can be coupled with a recorded signal or a pacing site. However, the accuracy of 3D-maps acquired by impedance recording only may be limited. It may be improved by combination with other modalities.

In conjunction with x-ray, radiopaque electrodes or markers (i.e. structures which are not used for signal acquisition or other electrical methods) may be applied at the applicator or another portion of the catheter. X-ray images can be combined with an electro-anatomical map for improving accuracy. Biplane fluoroscopy can be used for creating 3D geometry also without combination with electro-anatomic mapping.

Alternatively, magnetic coils may be applied to the catheter for electro-anatomic navigation. Furthermore, ultrasound sensors may be attached to the catheter for improvement of 3D registration.

The ablation device may further comprise a rotation limiting mechanism adapted to limit rotation of the first proximate end portion to a predetermined angular range. Thereby, excessive deformation of the ablation applicator and related hazards (in particular the risk of breaking) can be prevented. The predetermined angular range may e.g. be from -360° to +360°, such as from -240° to +240°, such as from -180° to +180°, such as from -120° to +120°, such as from -90° to +90°, such as from -60° to +60°. This applies in particular to an ablation applicator having a shape corresponding to a single loop. A rotational angle of 0° corresponds to the position where the shape and size of the ablation applicator is not influenced by rotation, i.e. where the ablation applicator has its pre-defined (also referred to as natural) shape. It is specifically noted that the angular range may also be asymmetric, such as from -150° to +240°. For ablation applicators with shapes corresponding to more than one loop, such as a helix shape with two full turns or more, the predetermined angular range may be from -720° to +720°.

The ablation device may further comprise a rotation feedback mechanism adapted to provide a feedback indication to a user when the first proximate end portion is rotated by a predetermined amount. The predetermined amount may in particular correspond to an amount of rotation where further rotation may cause applicator deformation. The feedback indication may be selected from the group consisting of a mechanical indication, a haptic indication, a visual indication, an acoustic indication, and a display indication, e.g. on a cryoablation console display.

The ablation device may further comprise a rotation blocking mechanism adapted to block rotation of the first proximate end portion when the ablation device is operable in a predetermined state. The predetermined state may in particular be a state where the ablation device is operated to position the ablation applicator relative to an ablation region within a patient. In this state, the ablation applicator will usually be stretched to extend snugly beside or around the first catheter in order to reduce the overall cross-sectional area during positioning, e.g. within a vessel or cavity, and may therefore be prone to hazardous deformation if a rotational force is applied to the ablation applicator. This is prevented by the rotation blocking mechanism.

The rotation blocking mechanism may in particular be configured to fix the rotational position of the first proximate end portion such that a rotational force applied to the shape memory structure by the applicator tubing during assembly is evened out or compensated by an opposing rotational force applied to the shape memory structure by the first catheter. In other words, the rotation blocking mechanism fixes the rotational position of the first proximate end portion in such a way that stress on the shape-memory structure is minimized, i.e. such the ablation applicator takes on its natural shape.

The ablation device may further comprise a rotation locking mechanism adapted to selectively lock the rotational position of the first proximate end portion. The rotation locking mechanism allows a user to lock the rotational position of the first proximate end portion and thereby to fix the applicator size to the adjusted value. Accordingly, once the applicator size has been optimally adjusted, it can be assured that the applicator size does not change during operation.

The ablation device further comprises a handle housing the first proximate end portion and the second proximate end portion, the handle comprising a rotational member for applying the rotational force to the first proximate portion. The handle may preferably constitute the operational interface of the ablation device in the sense that the operator can control all or most functions of the ablation device via the handle. The rotational member may in particular be adapted to engage directly the first proximate end portion or with a further rotational member, such as a toothed wheel, mounted on the first proximate end portion.

The first catheter of the ablation device may comprise an outer layer formed of a biocompatible material having an elastic modulus that is equal to or larger than 1500 MPa, in particular larger than 1900 MPa. This can e.g. be achieved by forming the outer layer of the first catheter of Nylon 12, Rilsan Clear or a similar material.

Generally, the first catheter should be laid out such that it withstands at least the applied torque for changing the applicator size without being negatively affected. This means that there should not be any significant amount of deformation, squeezing, torsion or similar damage on the first catheter when it is rotated. At the same time, however, the first catheter has to fulfill the mechanical, functional and safety properties described herein. This can in particular be achieved by choosing a suitable material for the catheter tube and/or by strengthening the part of the catheter that is subjected to the greatest mechanical load. In particular, the selected material should enhance the pushability and torque of the catheter while preserving flexibility and atraumatic properties. In some cases, a tapered catheter tube (having a larger cross-section towards the first proximate end portion and a smaller cross-section towards the first distal end portion) can be advantageous.

The ablation applicator may preferably be a cryo-ablation applicator. Thus, the ablation applicator may comprise an inner lumen serving as a boiling chamber for a cryogenic medium, such as nitrogen, supplied and drained by the second catheter.

The ablation applicator may preferably be designed such that, independent of its adjusted shape and size, it can apply a contact force to the to be ablated tissue that is similar to the contact force applied by a known (not adjustable) ablation applicator. In particular, the contact force does not change by more than 20% when the ablation applicator size is varied from its minimum size to its maximum size.

The ablation system may further comprise an electrical control unit adapted to communicate with the plurality of electrodes. The electrical control unit may in particular be adapted to apply predetermined electrical signals to selected electrodes and/or to obtain sensed electrical signals from selected electrodes and/or to process such sensed electrical signals.

Hereinafter, the invention will now be described in further detail by way of reference to examples of embodiment to which the invention is, however, not limited.

Fig. 1A shows a cross-sectional view of a heart.

Fig. 1B shows a loop-shaped ablation applicator arranged around the ostium of the left superior pulmonary vein.

Fig. 1C shows a set of lesions caused by an ablation applicator.

Fig. 1D shows a cross-sectional section of a cryo-ablation applicator.

Figs. 2A-2C show different configurations of an ablation device according to an exemplary embodiment.

Fig. 3A and Fig. 3B show configurations of an ablation device according to an exemplary embodiment.

Fig. 4A and Fig. 4B show configurations of an ablation device according to an exemplary embodiment.

Fig. 5A and Fig. 5B show an ablation device comprising a shape-memory structure according to an exemplary embodiment.

Figs. 6A-6C illustrate how rotation may cause hazardous deformation of an ablation applicator according to an exemplary embodiment.

Fig. 6D shows a rotation blocking mechanism according to an exemplary embodiment.

Figs. 7A and 7B show stress-strain curves for a shape-memory material in accordance with exemplary embodiments.

Figs. 8A-8C further illustrate how rotation may cause hazardous deformation of an ablation applicator according to an exemplary embodiment.

Fig. 8D shows a rotation blocking mechanism according to an exemplary embodiment.

Figs. 9A-9C show ablation devices according to further exemplary embodiments.

Figs. 10A and 10B show a rotation mechanism and a rotation blocking mechanism according to an exemplary embodiment.

Fig. 11 shows a refrigerant stopping structure in accordance with an exemplary embodiment.

The illustrations in the drawings are schematic. In different drawings, similar or identical elements are provided with the same reference signs.

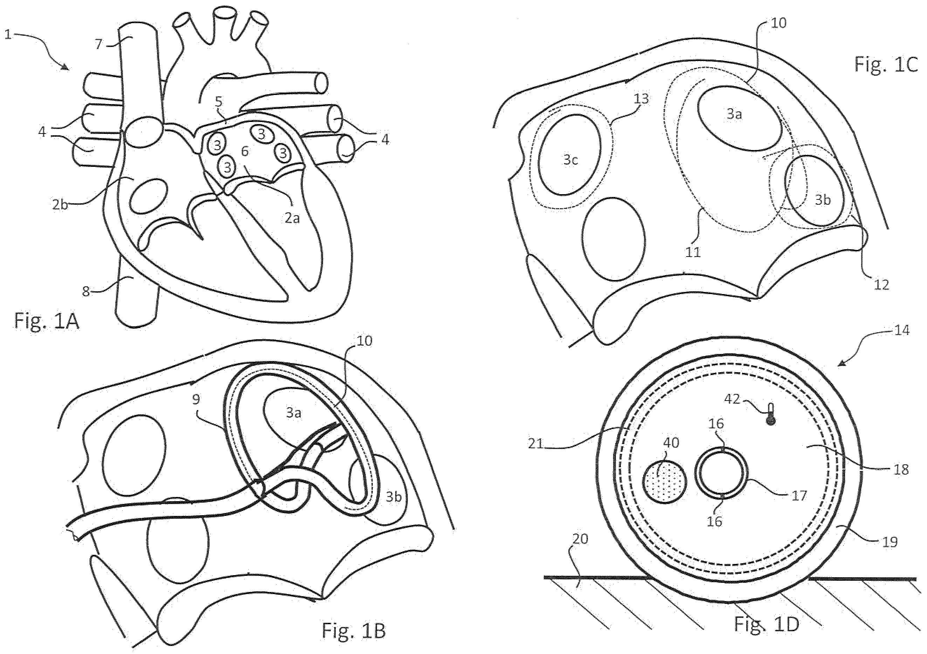

Referring now to **Fig. 1A**, a cross section of a heart **1** is shown. For treatment of a supraventricular tachycardia, such as atrial fibrillation, it is of particular interest to ablate tissue in a left atrium **2** of the heart **1**. Here, ablation may be performed particularly at or around the ostia **3** of the pulmonary vein vessels **4** of the heart. While Fig. 1A shows four such vessel ostia **3**, individual variation in humans may result in only three ostia in certain individuals and up to six ostia in other individuals. However, ablation therapy may also be performed in animals, such as e.g. horses, and the number of ostia may also vary from species to species.

In complicated types of atrial fibrillation or supraventricular tachyarrhythmia, ablation may not be restricted to the vicinity of the pulmonary veins **4**, but may be conducted also in other atrial tissue structures, such as for example the roof **5** or posterior wall **6** of the left atrium **2a,** or for example around the superior **7** or inferior **8** caval vein in the right atrium **2b.**

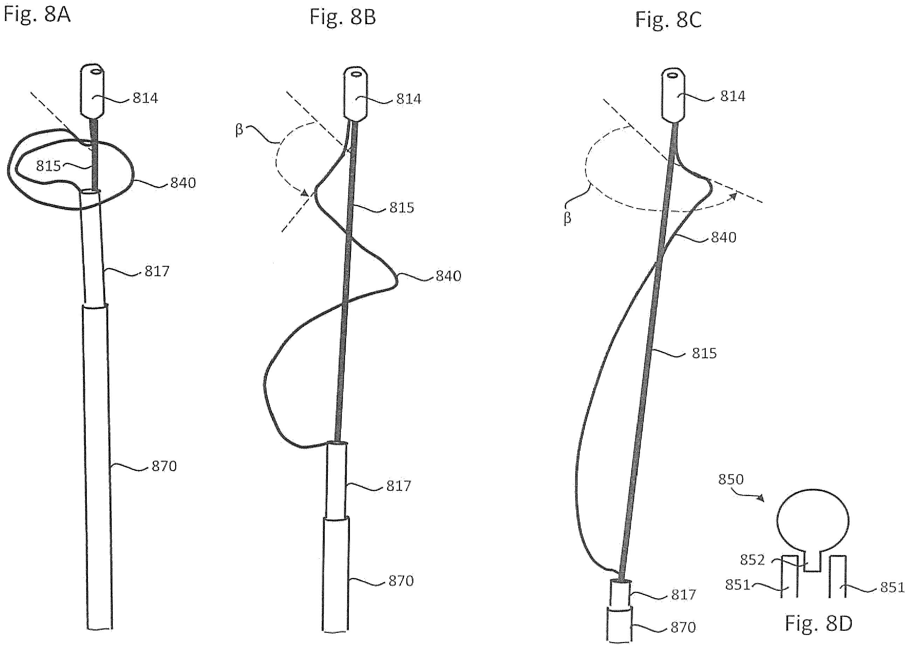

As an example, **Fig. 1B** shows a loop shaped catheter **9** placed around the ostium **3a** of the left superior pulmonary vein. As will be described in more detail below in conjunction with Fig. 8, the catheter **9** may be introduced into the body in a stretched configuration inside a sheath. A guide wire (not visible in Fig. 1B) may be used for placing the catheter tip into the pulmonary vein. For the depicted example, the loop **9** is opened such that it overlaps also with the ostium **3b** of the left inferior pulmonary vein. Along a therapeutically active segment of the loop, a lesion **10** as indicated by a dashed line may be created.

It may be desirable to be able to control the shape and size of the loop **9** such that, in a particular situation, it can be selectively enlarged for overlapping with two or more veins. In another situation, the loop diameter may be reduced for encircling a single vein. Within the scope of the invention, embodiments are presented which allow for such variation of shape and size of a therapeutically active segment of the catheter (also referred to as the ablation applicator).

**Fig. 1C** exemplarily shows a set of lesions created by multiple ablations. Lesion **10** is the lesion created by the exemplary configuration shown in Fig. 1B. Lesion **11** is created by rotating the catheter **9** in Fig. 1B by approximately 180° while leaving the size of the loop essentially unchanged. Note that lesions **10** and **11** in combination encircle and define a closed area. The pulmonary vein ostium **3a** is entirely enclosed by these two lesions **10**, **11**. The pulmonary vein ostium **3b** is partially encircled by the two lesions **10, 11**. Around the vein ostium **3b, 3c**, other lesions **12, 13** are created by placing the catheter at the ostium of the respective vein and reducing the loop diameter such that a single lesion completely encircles the corresponding pulmonary vein ostium **3b, 3c.**

Different types of ablation energy, such as radio frequency electric current, laser or cryo energy may be used to create lesions **10, 11, 12, 13** as those shown in Fig. 1C.

**Fig. 1D** shows a cross-section across a cryo-ablation catheter for the creation of an elongated pre-shaped lesion, i.e. a cryo-applicator **14**. The refrigerant supply line **15** guides liquid pressurized refrigerant to the therapeutically active segment (e.g. **9** in Fig. 1B). Refrigerant outlets **16** allow for passing the refrigerant from the supply line **17** to the boiling volume **18** of the cryo-applicator **14**. Note the cross-sections chosen in Fig. 1D. The outlets **16** have the smallest diameter or cross-section in order to allow for a distinct pressure drop from the supply line **17** to the boiling chamber **18**. This pressure drop allows for using the Joule-Thompson effect for reducing the temperature in the boiling volume to a freezing temperature range. The cross-section of the boiling volume **18** is significantly larger compared to the cross-section of the outlets **16** and the supply line **17** for maintaining the boiling volume **18** at a low pressure and thus at low temperature. Note that the boiling volume **18** is also used for draining the refrigerant from the cryo-applicator. In other words, the supply-line **17** and boiling volume **18** deliver and drain the refrigerant in a co-axial fashion. The outlets **16** spaced along the therapeutically active region provide a by-pass from the supply **15** to the boiling lumen **18**. Note that the boiling lumen **18** is also the drain pathway. A shape memory structure **40** is located inside the boiling lumen **18**. Furthermore, a temperature sensor **42,** such as a thermocouple or a thermistor, may be located inside the boiling volume **18**. In certain embodiments, a plurality of temperature sensors **42** may be used along the loop **9**. At the very distal end, the supply line **17** may be closed or a diameter reduction structure (not shown) may be inserted. While at least a significant portion of the refrigerant inside the supply line **17** may be in its liquid phase for cryo-ablation, a significant portion of the refrigerant in the boiling volume **18** may be in the gaseous phase.

The boiling volume **18** is surrounded by an applicator tubing **19** which has a double function. Firstly, it seals the boiling volume **18** from the surrounding body environment (blood flow, tissue) and, thus, it provides a significant contribution to the safety of the device. Secondly, it provides the heat transfer from the boiling volume **18** to target tissue **20** and, thus, provides a significant contribution to the therapeutic effect of the device.

A support structure **21** is foreseen for avoiding kinking of the applicator tubing **19**. This support structure **21** may be a helical coil or a wire-frame structure. A shape memory component may be used for selecting a predefined shape of the ablation structure.

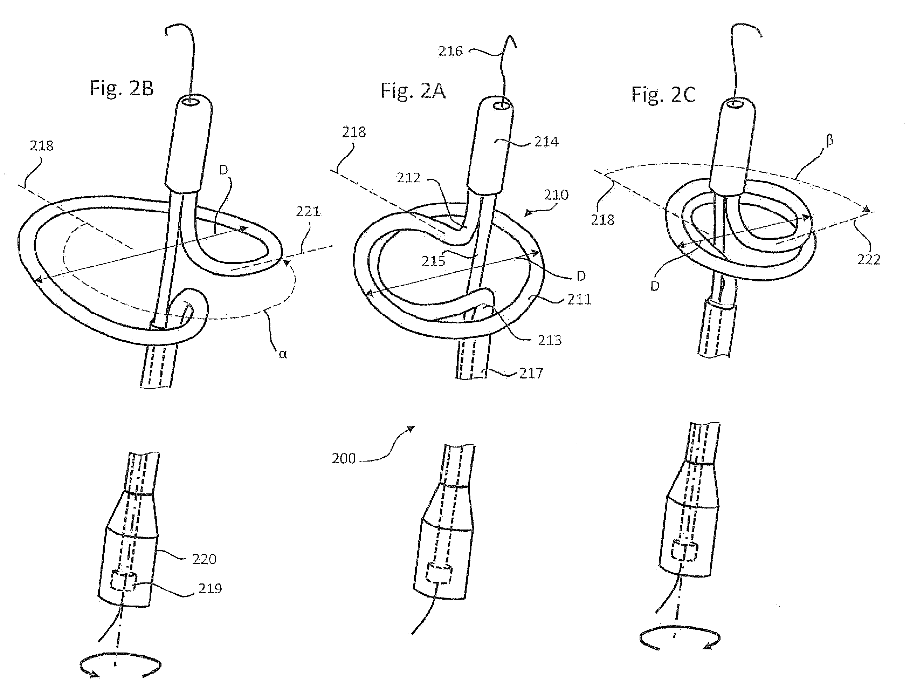

An example embodiment of a cryo-ablation device **200** is shown in **Fig. 2A**. An essentially circular cryo-ablation applicator **210** forms a therapeutic segment (elongate portion) **211** which allows for ablating approximately one full turn of a circle by cryo-ablation. At the distal end, a curved connective segment (first applicator end portion) **212** mechanically connects the cryo-applicator to a tip-structure **214** of the catheter. This tip structure **214** hosts also a positioning catheter **215**. This positioning catheter hosts also a lumen for a guide wire **216** which may be used for positioning the tip **214**, e.g. inside the ostium of a vessel. Alternatively, instead of a guide wire, also an inner lumen lasso catheter may be placed inside the positioning lumen. For inserting the catheter into the body, the positioning catheter **215** may be used for stretching the cryo-loop structure **210** to an essentially straight but precoiled configuration. Once the catheter is inserted into a particular body region, such as a target chamber of the heart, the positioning catheter **215** may be pulled back for restoring the essentially circular shape of the applicator **210**.

On the proximal end of the cryo-applicator **210**, a second pre-curved connecting segment (second applicator end portion) **213** is used for connecting the cryo-applicator **210** with the ablation catheter (or shaft) **217.** This connection has a double function. Firstly, it mechanically attaches the cryo-applicator **210** to the shaft **217**. Secondly, it establishes a co-axial refrigerant pathway from the shaft **217** to the applicator **210** for supply and draining of refrigerant.

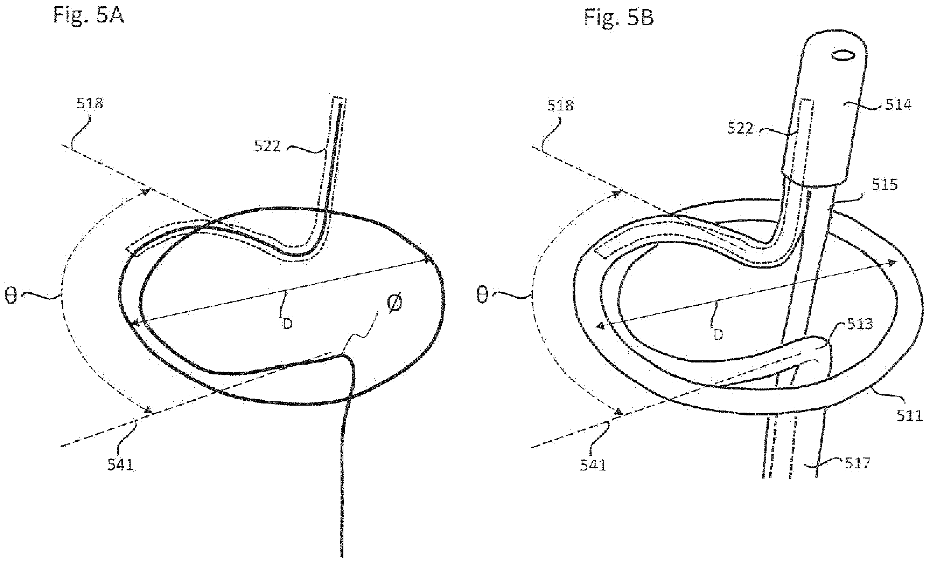

The shape of the loop **211** and the two connective segments **212, 213** may be predefined by a shape memory component as shown in Fig. 1D. Note that when inserting a shape memory component into the applicator tubing **19** and the support structure **21**, due to the stiffness of components, the resulting diameter of the shape memory component inside the loop **211** may be somewhat larger than its original diameter as some force is needed to bend structures **212, 213**. As a consequence, such an increased diameter will go hand in hand which a mechanical pretension in the shape memory component. As will be shown in Figs. 5A and 5B, it may be beneficial to reduce this pretension inside the shape memory component to an acceptable low value. This can be done by fixing the loop-structure **211** at the distal tip connection and proximal shaft connection during manufacture of the device **200** in such a fashion that the diameter of the shape memory component inside the device is close to its original diameter. Such a configuration, which takes advantage of pre-mounting the shape memory structure with low pre-tension, is called the natural shape configuration. A hashed line **218** marks the azimuthal orientation of the distal connective segment **212**.

**Fig. 2B** shows an example for increasing the loop diameter **D** by rotating the positioning catheter **215** relative to the shaft **217**. A torque transmission structure **219** is attached to the proximal end of the positioning catheter **215.** In one embodiment, the torque transmission structure **219** may be a gear wheel or cog wheel used in combination with a gear mechanism inside the handle **220** which allows for adjustment of size by a rotational mechanism (not shown) included in the handle **220**. However, in another embodiment it could also be a grip piece attached to the positioning catheter which extends outside the handle **220** and may be rotated by hand.

The torque applied to the proximal end of the positioning catheter is transferred to the distal end of the positioning catheter and, thus, to the tip structure **214**. Thus, the tip **214** and the distal end **212** of the cryo-applicator **210** are rotated. For the example shown in Fig. 2B, the size D of the loop **211** is increased by turning the distal connective segment **212** by an angle $\alpha$. Thereby, the first applicator end portion **212** is rotated from position **218** to position **221**. Note that in this application, the tip **214** must fulfill multiple tasks. Firstly, it must transmit all translational and rotational forces needed for stretching and rotating the loop **211**. Secondly, it must also provide a reliable distal sealing of the boiling volume. This sealing must remain mechanically intact even at the described translational and rotational forces and at the given operating pressures of the cryoablation catheter device.

The torque applied by the operator will be transferred to the tip. For avoiding damage of material by the forces or torque applied for shape and/or size adjustment of the device, deformations should be carried out within the elastic or superelastic window for all materials that are used. Thus, a linear relation may be used for estimating the torque needed for rotating the tip for a given angle relative to the shaft:

$$\text{torque = torsional stiffness constant (loop property)} \times \text{angle of loop.}$$

For certain embodiments, the torsional stiffness constant of the loop structure **210** may be selected to be in within a range of 0.1 Nmm/rad to 10 Nmm/rad.

As the positioning catheter **215** transfers the torque to the tip **214**, the torque or rotational force applied to the proximal end **219** of the positioning catheter **215** will also cause some twisting of the positioning catheter **215** around its longitudinal axis. Therefore, the angle of rotation which has to be applied at the proximal end of the positioning catheter **215** is larger than the angle of rotation at the loop **211**. The angle of rotation at the proximal end of the positioning catheter **215** is the angle of rotation of the loop **211** plus the angle of twist of the positioning catheter **215**. For keeping the angle of twist of the positioning catheter **215** sufficiently small, the torsional stiffness of the positioning catheter **215**, or in other words the torsional stiffness constant of the positioning catheter **215**, should be small, too. Thus, the torsional stiffness constant of the positioning catheter **215** should be at least 70% the torsional stiffness constant of the loop **211** or, more generally, at least 100% of the torsional stiffness constant of the loop **211**.

Referring now to **Fig. 2C**, the positioning catheter **215** is rotated by an angle $\beta$ in the opposite direction in comparison to Fig. 2B. Thereby, the position of the first applicator end portion **212** is rotated from position **218** to position **222,** and the size D of the loop **211** is decreased. Note that for the embodiments shown in Figs. 2A to 2C, the helix pitch is a left hand screw. Obviously, also a right hand screw geometry may be used mirroring the orientation of rotation for increase / decrease of loop size D.

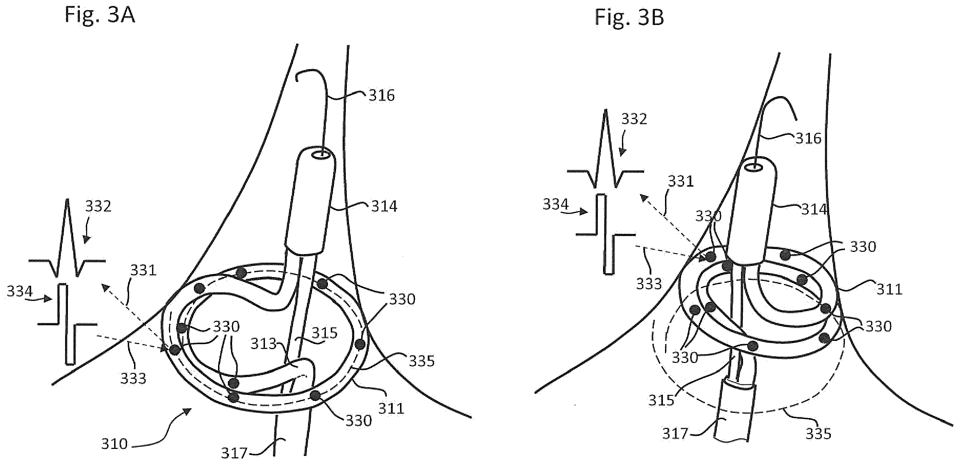

Referring now to **Fig. 3A**, a plurality of electrodes **330** are provided on the surface of the loop **311** of the cryo-applicator **310**. The electrodes **330** may be surface printed flexible or stretchable electronic structures, as depicted in the figure. However, in different embodiments also conventional ring electrodes made from platinum, platinum iridium, gold or any other suitable metal may be used. These electrodes can be used for multiple purposes:
Electrodes **330** may be used for sensing signals from the tissue, as described in the art. This is depicted by an arrow **331** indicating that the signal **332** is sampled from the tissue by an electrode **330**.

Furthermore, electrodes **330** may be used for pacing the tissue as described in the art. This is depicted by an arrow **333** indicating that a stimulation pulse **334** is transferred to the tissue by an electrode **330**.

Furthermore, electrodes **330** may be used for impedance measurements. In such a situation, high frequency electric currents are applied by the electrodes **330**. These currents can be used for assessing wall contact or ice-formation on the cryo-applicator, as described in the art, for guiding the ablation process.

Furthermore, impedance measurements may be used for coupling the catheter electrodes **330** with impedance based electro-anatomical mapping systems, as described in the art (see e.g. WO 2013/039564 and US 7.263.397). In some applications, the accuracy of electro-anatomical mapping may be increased by combination with magnetic sensors (see e.g. US 2013/006194) and ultrasound sensors (see e.g. US 8 428 691) on the catheter device.

An essentially circular lesion **335** or a lesion set as shown in Fig. 1C may for example be created by cryo-ablation and the electrodes **330** may be used for assessing the outcome of the ablation process after the delivery of ablative energy, either by recording of intracardiac signals or by pacing, i.e. by the stimulation of tissue using electric pulses. As it is shown in Fig. 3A, this may be done by maintaining the catheter in the very same position as for ablation. In such a situation, the catheter remains located directly at the lesion **335** (i.e. at the border of the isolated area). If the diagnostic procedure is performed with a similar geometrical shape as the preceding ablation step, diagnostic investigation may be carried out directly at the boarder of viable and ablated tissue. This may reduce the accuracy of the investigation.

In **Fig. 3B,** the size of the loop **311** is reduced for assessing the outcome of ablation inside the lesion. An electrode **330** may be used for assessing whether electrical activity from the tissue outside the lesion (paced or spontaneous activity) can enter the region from beyond the tissue (entry block achieved or not?). For assessing entry block, one or a plurality of electrodes **330** at the loop **311** of reduced size may be used for sensing activity inside the lesion. Note that by sensing at a certain distance from the lesion, sensitivity to far field potentials may be reduced yielding a more reliable assessment of entry block.

Furthermore, if a lesion encircling a region is continuous, stimulation of tissue inside the region will not result in any activity in the tissue outside the region (exit block) as it can be assessed by body surface electrocardiogram or standard diagnostic electrophysiology catheters. Thus, pacing at one or a plurality of electrodes **330** at the loop **311** of reduced size (Fig. 3B) may be used for assessing exit block. Note that pacing at a certain distance inside the lesion may reduce the probability of unintended stimulation of the tissue outside the lesion. This may allow for a more reliably verification of exit block.

Note that the use of size adjustment for the verification of entry and exit block verification is not limited to the decrease of size as shown in Fig. 3B. By increasing the size, as shown in Fig. 2B, one or a plurality of electrodes **330** can be used for sensing and/or pacing of the tissue outside the lesion **335**. Any standard diagnostic catheter, as for example an inner lumen lasso catheter or standard linear multipolar diagnostic catheter, can be used for the simultaneous pacing and/or measurement inside the lesion **335.**

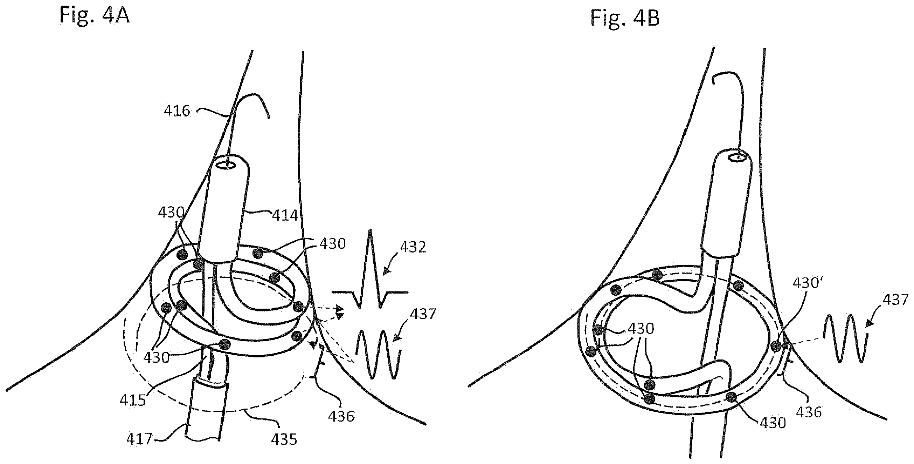

Referring now to **Fig. 4A,** a situation is depicted where the assessment of entry or exit block reveals an incomplete lesion, such that further treatment is required. After a first ablation as described above in conjunction with Fig. 3A, a gap **436** may occur within the lesion **435.** This means that after ablation, there is still some viable and electrically conducting tissue within a segment **436** of the lesion **435.** As a consequence, when testing for entry block, signals **432** recorded in electrodes **430** adjacent to the gap **436** may be of a relatively large amplitude or relatively early activation time. When testing for exit block, pacing at electrodes **430** near the gap **436** may result in fastest conduction from "inside to outside" or only at electrodes **430** adjacent to the gap **436,** activity may activate the tissue outside the target region of ablation.

Multiple strategies may be applied for closing the gap **436.** For example, the catheter may be shaped back to its original configuration (i.e. similar to that in Fig. 3A) and ablation may simply be repeated. Additionally or alternatively, the catheter may be rotated by a distinct angle (for example 45° to 135°) around its longitudinal axis and the ablation may be repeated with a different azimuthal orientation of the loop **411.**

Referring now to **Fig. 4B,** another strategy is presented which may be applied alternatively or additionally. As shown, the catheter is shaped back to its original configuration and the location of electrode **430'** may approximately mark the location of the lesion gap **436.** For selectively ablating the tissue only at this site, radio frequency current of sufficient strength may be applied via the electrode **430'.** For the current return path, a standard RF body surface patch electrode may be used or a second electrode **430** on the loop **411** may be used for defining the return path (bi-polar RF ablation). Alternatively, a plurality of electrodes **430** may be used in parallel for the delivery or return path.

As a particular embodiment, a double mode ablation catheter may be provided. The standard mode may be used for creating an elongated lesion by cryo-ablation. For the standard mode, the even distribution of refrigerant along the active cryo-segment of the catheter allows for effective creation of elongated lesions. The selective closure of small remaining gaps (touch-up ablation) may be performed using radio frequency ablation (touch-up mode). As heating of surface electrodes by radio frequency ablation may be potentially thrombogenic or may cause thermal stress to the electrodes on the loop, overheating may be prevented by operating the cryo-pathways of the catheter at a reduced cooling power. This means that a cryo-console controlling the refrigerant flow to the catheter may deliver the refrigerant at a reduced flow rate or at reduced cooling capacity (for example, the refrigerant may be delivered to the supply lines at least partially in its gaseous phase or at a pressure below the nominal working pressure for cryo-ablation). Note that such a cooling mode, which prevents the electrodes from overheating, differs from standard cryo-ablation. Bio-physically, there is a comparable scope to irrigation of radio frequency (flushing with saline solution). However, different from saline irrigation, here a sealed cooling pathway is used including Joule-Thompson effect based temperature reduction.

The embodiments shown in Figs. 3A, 3B, 4A, and 4B may be modified to be used only for diagnostic purposes, i.e. to leave out all features relating specifically to cryo-ablation.

Referring now to **Fig. 5A**, a shape memory component **540** (see also Fig. 1D) is shown with its predefined shape. For the depicted example, the shape memory component **540** is a wire with a defined geometrical shape which has been defined by a dedicated shape definition formation process, such as heat treatment or plastic deformation. The use of super-elastic materials, such as Nitinol or cooper-aluminum-nickel alloys, other shape memory alloys or shape memory polymers, may be beneficial as they allow for a relatively large deformation when navigating the catheter device along the vasculature of a human or animal body. However, in certain applications, it may be sufficient to allow only moderate deformations and material such as stainless steel or a comparable material may be used. Fig. 5A shows the geometry of the shape memory component **540** after the shape definition process at room or normal ambient temperature (e.g. at 10°C to 40°C). Two parameters defining the shape of the structure are indicated: the size D and the angle θ between the two connective segments **518**, **541.**

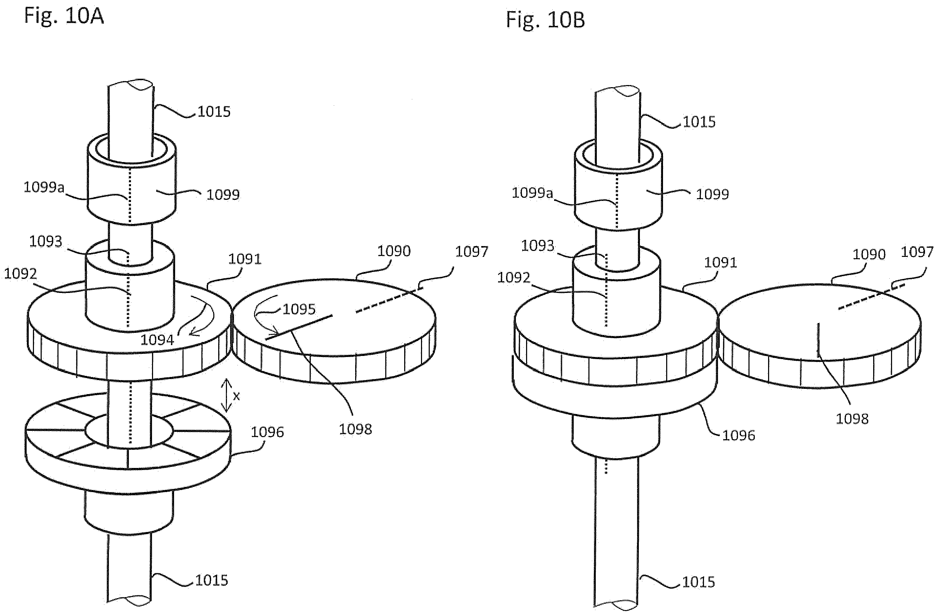

In **Fig. 5B**, the shape memory wire **540** is mounted onto the catheter assembly. This means that it is enclosed inside the cryo-applicator tubing **511, 512, 513, 517** (see also Fig. 1D) and therefore not visible from outside. However, the applicator tubing is mounted in such a fashion on the catheter device, that the shape of the wire **540** inside the applicator is essentially the same as after shape definition in Fig. 5A. Thus, for adjusting the size of the loop **511** to its natural shape the rotational position of the positioning catheter **515** is set to a predefined angle as described in more detail in Figs. 10A and 10B. Note that the two parameters size D and angle θ are essentially the same in **Fig. 5A** and **Fig. 5B.** Thus, the applicator tubing **511, 512, 513, 517** does not translate a noticeable mechanical stress on the shape memory competent **540**. In other words, any avoidable pretension on the shape memory component is reduced to a small level. Such a configuration may be referred to by the term "natural shape configuration."

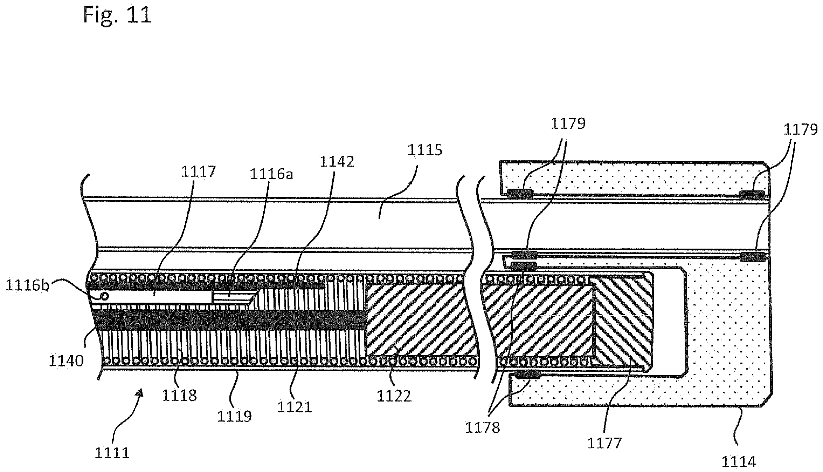

As shown in Figs. 5A and 5B, a refrigerant stopping tube or structure **522** may be foreseen on a distal portion of the shape memory wire **540**. This refrigerant stopping structure **522** will be described in more detail in Fig. 11. The refrigerant stopping tube **522** may be placed concentrically over the shape memory component **540**. Tubing **522** may be made from a soft material as for example a plastic of a low shore hardness such that its bends to the natural shape of the wire **540** at a low force.

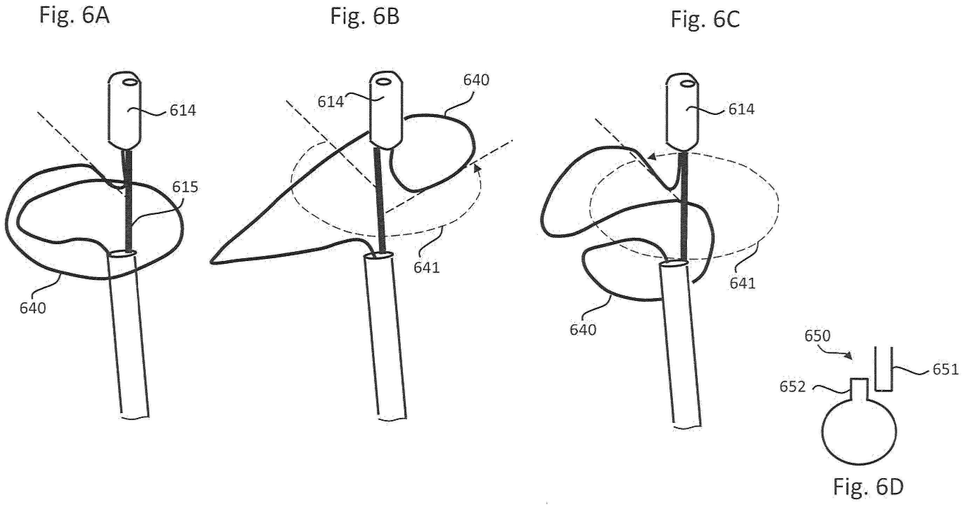

When altering size or shape of a of cryo-applicator structure by a torsional or rotational mechanism, deformation must remain within certain limits for ensuring safe operation of the catheter device as it is outlined in **Figs. 6A to 6C.** **Fig. 6A** shows the catheter in a natural shape configuration. For illustration purposes, the cryo-applicator tubing is not shown in the image. The only loop component shown is the shape memory structure **640** (i.e. also the support structure, the supply line and the thermo-couple are not shown for making the shape memory component visible). Note that in this example, the shape memory component **640** is a helix with approximately one full turn.

In **Fig. 6B**, the size of the loop is increased by turning the tip **614** by almost one full rotation (i.e. by turning it almost by the same angle as the single turn contained in the natural shape). By turning the tip **614,** a torque or rotational force is transmitted to the wire **640** as indicated by arrow **641** in Fig. 6B. As a consequence, the shape memory structure **640** is twisted around its longitudinal axis. When turning the tip **614** by a critical angle (almost one turn for the given example), the shape memory wire **640** in the middle of the loop becomes almost straight as indicated by arrow **641** marking a critical deformation of the loop.

In **Fig. 6C,** the tip **614** is further turned beyond the critical deformation shown in Fig. 6B. As consequence, the shape memory wire **640** is further twisted around its longitudinal axis and the loop degenerates to a geometry containing two branches of opposite curvature. Such a degenerated geometry may not allow for a controlled contact with tissue and may induce a considerable mechanical stress not only in the shape memory component but also in its surrounding structures, such as the cryo-applicator tubing (not shown). Having in mind that the cryo-applicator tubing is also the sealing safety structure enclosing the boiling volume, mechanical stress in the tube must be limited to an acceptable value. In the worst case, high stress may cause fatal damages, such as micro fractures to the tubing, and refrigerant may escape into the body during freezing. Therefore, only a maximal angle of relative rotation may be allowed. **Fig. 6D** exemplarily shows a simple stopper mechanism **650** for defining such a maximum angle of deformation. Here, rotation beyond a certain angle is prevented by stopper **651** which does not allow protrusion **652** to rotate beyond a certain point. Note that Fig. 6D is included for purposes of illustration only and that various stopper mechanisms, as known in the art, may be used.

While Figs. 6A to 6C refer to deformations induced by increasing size, a twist of opposite orientation is induced by decreasing size. Thus, two stoppers may be used, i.e. one defining the maximal allowed degree of increase and the other one defining the maximal degree of decrease.

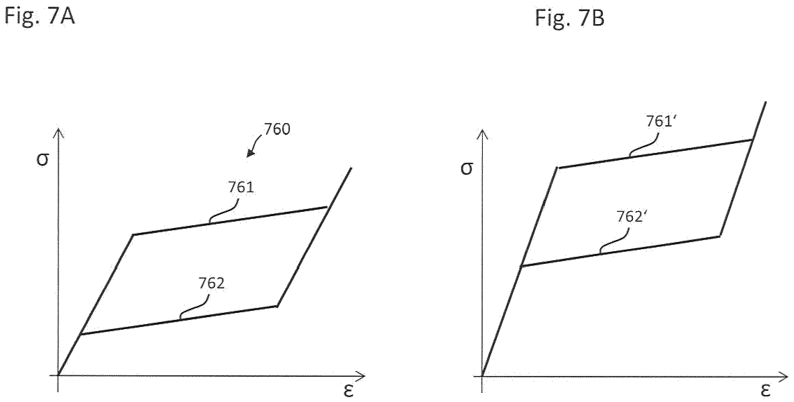

Referring now to **Fig. 7A,** an example of a stress-strain curve **760** for a shape memory alloy at essentially body temperature (e.g. 30°C to 45°C) is given. When deforming a shape memory material, for example by pulling in an initial phase, an approximately linear increase of stress σ is induced by low strain (elastic behavior). Above the elastic limit of the material, a small increase of stress σ will result in a large elongation ε of the material. In other words, a considerable increasing deformation of the material can be observed at an almost constant upper plateau stress level **761** (super-elastic deformation). When releasing the forces causing deformation, the material recovers to its original shape only at a reduced lower plateau stress level **762**. This well-known behavior of many shape memory alloys means that some shape-memory alloys deliver only moderate forces when restoring their shape after a release of force.

A small value of lower plateau level stress **762** may be a noticeable disadvantage for the described methods of shape adjustment. When adjusting shape in a wide range, deformation will typically occur within the super-elastic domain. However, this may hamper the catheter device from returning to its original shape as the restoring forces may be rather small. Small restoring forces may be insufficient for promoting strain release in the presence of unavoidable competing forces, such as friction or anatomical obstacles.

For ensuring that a deformed cryo-applicator structure can always return to its natural shape, it may be advantageous that the lower plateau level stress **762'** is sufficiently high, as shown in Fig. 7B. For example, when using nitinol as a shape memory alloy, there are several options for influencing the lower plateau level stress. Firstly, heat treatment impacts both the upper and lower plateau stress levels. As a rule of thumb, decreasing of the Austenite finish temperature of Nitinol by proper heat treatment increases both plateau levels **761', 762'.** Furthermore, particular Nitinol alloys, such as Chrome dotted Nitinol can be used for increasing both plateau levels **761', 762'.**

Thus, the material and its heat treatment may be selected such that the loading plateau **761'** at body temperature may be at least at 450 N/mm$^2$ or more, particularly at least at 550 N/mm$^2$. Furthermore, the material and its heat treatment may be selected such that the unloading plateau **762'** may be at least one third of the loading plateau level **761',** or more particularly at least one half of the loading plateau level **761'.**

Referring now to **Fig. 8A,** a catheter is shown in its natural shape configuration. Similar to Figs. 6A to 6C, the shape memory component **840** is made visible by hiding the applicator tubing and other surrounding structures, such as support structure, refrigerant supply line and thermocouple in the illustration. At the distal portion of the catheter, the positioning catheter **815** and the pre-shaped portion are connected to the tip **814** of the catheter. For the shown example, the loop is essentially in its natural configuration. For some applications, the catheter shaft **817** may be guided within a sheath **870** for maneuvering the catheter inside a body. For insertion of the catheter into the body and for removal from the body, the pre-shaped structure **840** may be stretched to an essentially straight configuration.

As shown in **Fig. 8B,** by advancing the positioning catheter **815** in a distal direction relative to the shaft **817,** the shape memory component **840** is stretched. Stretching of the shape memory component involves bending of the shape memory component away from its natural stored shape. This bending creates a reaction torque which tends to "open" the helix by rotating the tip corresponding to an angle β. As show in **Fig. 8C,** further stretching further increases the angle β. For the configuration shown in Figs. 8A to 8C, the pull force applied for stretching the loop will induce a torque in the shape memory component **840** which in turn induces rotation of the tip portion **814.** Rotation of the tip **814** induces a twist of the positioning catheter **815.** This twist within the position catheter **815** in turn provides a counter torque which tends to reduce the angle β. In other words, the higher the torsional stiffness of the positioning catheter, the smaller the twist angle β observed at the distal tip will be.

For the shown example, it is assumed that the proximal end of the positioning catheter **815** is kept at a relative angle which corresponds to the angle of the natural configuration of the catheter. A guiding structure **850** ensures that this angle may be maintained during stretching of the device by advancing the positioning catheter. Here, guiding elements **851** do not allow protrusion **852** to rotate beyond a narrow interval. Such a structure for preserving the proximal rotational angle during stretching may be highly advantageous. As it has been shown in Figs. 6A to 6C, excessive rotation of the tip **814** may lead to severe deformation of the loop which may cause damage to the device. When the device is in an essentially straight configuration, a very small amount of torque can rotate the tip beyond a safe acceptable maximum. In this context the "self-opening" tip rotation described in Figs. 8A to 8C is a safety critical property of a stretched loop configuration. For ensuring functional safety, two measures should be taken. A sufficiently large bending stiffness may be assigned to the positioning catheter **815** and its proximal end may be fixed at a rotational angle which corresponds to its angle in the natural loop configuration.

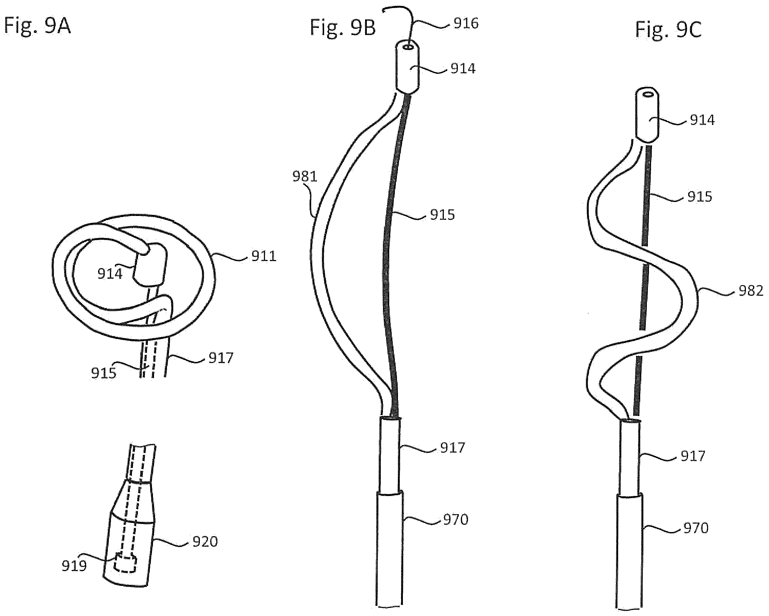

In **Fig. 9A**, another example embodiment is shown. In this configuration, the torque transmitting structure **914** does not protrude distally from the loop plane. Such a configuration may be used for a variety of applications, where ablation is not performed at a vessel ostium. For example, such a configuration may be applied at the posterior atrial wall or at the roof of the left atrium. In other words, it may be applied in situations where anchoring of a catheter tip at a vessel is not possible or not needed. Therefore, such a configuration may be applied for a broader scope of organs, such as e.g. kidney or liver.

As another example, a curved cryo-applicator segment **981** is shown in **Fig. 9B.** For example, such a configuration may be used for creating an elongated ablation line along a posterior wall of the roof or a lateral wall of an atrium or any other body cavity. The shape memory component (not shown) inside the applicator **981** may be trained to a natural geometry. For the example shown in Fig. 9B, this natural shape is essentially a segment of an arch lying in a plane. Note that the natural shape is obtained by advancing the positioning catheter **915** to the depicted "intermediate position". By further advancing the positioning catheter tip **914** to a more distal position, the arch may be stretched (in other words, its curvature may be reduced). By pulling back the positioning catheter **915**, the cryo-applicator may bend back to its natural shape (in other words, its curvature may be increased). By rotating the tip **914** in either direction, the curve of the arch **981** can be modified such that it is curved in all three dimensions (i.e. not lying in a plane anymore). Such a broad variety of curves can be realized. Therefore, the application of this configuration may be of interest also for vessels, such as renal arteries or an aorta. For example, the cortex epileptic tissue or a tumor may be treated by such a configuration.

As it is shown in Fig. 9B, also the positioning catheter **914** may slightly bend in the natural shape. This is due to the fact that some force and/or torque is needed for bending the applicator tubing and the support structure to the natural shape. For handling these forces, it is desirable that the positioning catheter **915** provides a relatively high bending stiffness. Some manufacturing techniques applied for increasing the torsional stiffness, such as braiding or the use of high durometer plastics, may be used for altering also the bending stiffness of the positioning catheter **915**. In addition to that, a guide wire with a high distal bending stiffness may be used for providing sufficient mechanical stiffness for adjusting the shape of the curved arch **981** for matching the shape of the tissue.

Another example of a pre-shaped curved arch cryo-applicator **982** in its natural shape is shown in **Fig. 9C.** In this configuration, the pre-shaped memory component is not lying within a plane but curved into all three dimensions. Such a configuration may be used for creating shapes which cannot be obtained by the configuration shown in Fig. 9B. Also here, longitudinal displacement of the positioning catheter **915** in combination with rotation of tip **914** and/or shaft **917** can be used for obtaining a desired shape.

In **Figs. 10A and 10B,** some example locking mechanisms are presented which may be foreseen within the handle of a catheter (not shown). When adjusting the shape of the loop by rotational or translational movement, it may be desirable to mechanically fix an adjusted shape. This may be accomplished by mechanically fixing the relative rotational and translational position of the positioning catheter **1015** relative to the shaft.

By turning a rotation control wheel **1090**, a gear mechanism can be used for translating the rotation or torque to a rotation adjustment wheel **1091**. Guiding structures **1092** and**1093** on the adjustment wheel **1091** and the position catheter **1015** may be used for translating the rotation of the adjustment wheel **1091** onto the positioning catheter **1015** while allowing the positional catheter **1015** to move axially relative to the adjustment wheel **1091.** For the depicted example, control **1090** and adjustment **1091** wheel are turned as indicated by arrows **1094** and **1095** by exemplarily about 45°. As can be seen from **Fig. 10B**, the control wheel **1090** is then rotated by approximately 45° in a counterclockwise direction while the adjustment wheel **1091** and the positioning catheter **1015** are rotated by approximately 45° in a clockwise direction.

For enabling and fixing of this rotation, a fixation disc **1096** may be provided. In Fig. 10A, this fixation disc **1096** is translationally detached from the adjustment wheel **1091** and rotation is enabled. In **Fig. 10B**, the fixation disk **1096** and the adjustment disk **1091** are attached to each other. The fixation disc **1096** is arranged in the handle (not shown) such that it is not allowed to rotate relative to the long axis of the catheter shaft. By fixing also the angular rotation of the shaft relative to the handle, also the positioning catheter **1015** is fixed in its actual angular position in Fig. 10B. Note that bearings (not shown) may be used for accommodating gear wheels, such as the adjustment **1091** or control **1090** wheel within the handle. In some embodiments, the friction of the gears may be sufficient for fixing the relative rotational orientation of the positioning catheter **1015.** In this situation, the use of a fixation disk or comparable structure may be avoided.

In some embodiments, additional translational gear wheels may be provided in addition to the adjustment **1091** and control **1090** wheel. In yet another embodiment, a single combined adjustment/rotation wheel may be provided.

As can be seen from Figs. 10A and 10B, a stopper structure **1097** may be foreseen. In combination with a counter stopper **1098** on the control wheel **1090**, the allowed angle of rotation may be limited. For the shown example, this mechanism allows for a maximal relative rotation by approximately 180° in either direction. However, by adjusting the gear ratio and stopping points, any other range of allowed rotation can be adjusted.

For the embodiment shown in Figs. 10A and 10B, also a translational guiding sleeve **1099** is foreseen. This guiding sleeve may be rigidly coupled to the handle. For the natural shape configuration shown in Fig. 10A, a guiding structure **1099a** inside the sleeve aligns with the guiding structure **1093** on the positioning catheter. Thus, the positioning catheter can be advanced for stretching the catheter while preserving the angular orientation of the proximal positioning catheter as described also in Figs. 8A to 8B. In Fig. 10B, the guiding structure **1099a** on the translational guiding sleeve **1099** does not align with the guiding structure **1093** of the positioning catheter **1015**. Thus, it is not possible to stretch the device when the positioning catheter is rotated to a different angle compared to the natural shape.

Referring now to **Fig. 11**, the distal connection of the cryo-applicator **1111** to the distal tip **1114** is shown. The outer applicator tubing **1119** is disposed on the support structure **1121**. Inside the boiling volume **1118,** the shape definition component **1140** is located. Refrigerant outlets **1116a** and **1116b** allow for passing the refrigerant from the supply tube **1117** to the boiling volume **1118**. This goes along with an expansion of the refrigerant due to a relatively large cross section of the boiling volume **1118**. The drop of pressure along the supply path **1117, 1116a** and **1116b** lowers temperature significantly below the freezing point. A temperature sensor **1142** may be foreseen for monitoring temperature inside the boiling volume **1118**. Proximally from the distal end of the refrigerant supply line, a refrigerant stopping tube **1122** is located on the shape definition component **1040**. The refrigerant stopping tube **1122** defines the distal end of the boiling volume **1118** as it occludes most of the cross-section inside the support structure **1121**. Note however, that the refrigerant stopping tube **1122** provides no perfect sealing to the distal end of the applicator as small gaps may remain between the refrigerant stopping structure **1122,** the support structure **1121** and the outer applicator tubing **1119.**

In order to provide a safe sealing of the distal end of the applicator, a sealing occlusion **1177** is provided at the distal end of the cryo-applicator **1111**. This sealing occlusion **1177** provides a tight connection with the outer applicator tubing **1119** preventing for the escape of any refrigerant out of the boiling chamber **1118**. The sealing occlusion may be created by a welding, molding or gluing process or the like. In certain embodiments also a mechanical seal may be provided.

As the proximal sealing occlusion is an important safety feature of the device, mechanical or thermal stress should be avoided for the occlusion structure **1177.** By keeping the gaps between the refrigerant stopping structure **1122,** the support structure **1121** and applicator tubing **1119** sufficiently tight, only a small amount of refrigerant may pass the gaps. Thus, there is only little cooling power and the refrigerant completely boils out and rewarms within the gas phase. Note that the refrigerant blocking structure **1122** is relatively long as can also be seen from Fig. 5A and 5B. Thus, no significant cooldown is observed at the occlusion structure **1177** during cryo-ablation.

Furthermore, the mechanical connection **1178** of the tip structure **1114** with the applicator tubing **1119** is provided somewhat proximally from the occlusion structure **1177.** Thus, force and/or torque transmission is applied to the applicator tubing **1119** without imposing serve stress to the occlusion structure **1177.** Also the connection **1178** of the applicator tubing **1119** to the tip **1114** may be provided by welding or gluing or comparable processes. Note that this connection provides a second sealing of the boiling volume **1118,** which acts as a back-up (complementary safety feature).

For transmitting forces (translational and/or rotational) via the positioning catheter **1115** to the tip **1114,** mechanical connections **1179** are foreseen. Also these connections may be provided by welding or gluing or comparable processes. Note that also these connections **1179** are crucial with respect to safety of the device as they secure the tip on the positioning catheter.

When altering the size of a loop shaped ablation applicator, e.g. as described in various ways above in conjunction with exemplary embodiments or in other ways falling within the scope of the present invention as set forth in the appended claims, it may become difficult to make and/or maintain contact with the tissue along the portion of the applicator which is used for ablation. Typically, a sheath is used for introducing the catheter into a body and for navigating it towards the target tissue. For improving tissue contact, an expandable balloon or dilatable member may be foreseen at a very distal portion of the sheath. As the sheath can be advanced relatively to the longitudinal axis, the balloon can be used for pressing the ablation catheter against the tissue. This may enhance tissue contact and improve the outcome of the ablation procedure. Furthermore, in addition to enhancing wall contact, a balloon may have a beneficial thermal effect on the ablation procedure.

Correlation between size change and rotation

On average, pulmonary veins of adult hearts have a diameter of about 10 to 25 mm. In general, the diameter of the applicator loop should be at least as big as or bigger than the diameter of the vein in the heart to be treated. As a non-limiting example this can be 20mm or more for a typical pulmonary vein of an adult heart. More preferably, the possible enlargement of the diameter should allow the diameter to reach up to double the vein size for getting good wall contact without vein injury, for measuring electrical signals in the vein and for freezing a common ostium. Adjusting the loop size should be achievable within a short period of time. In addition, the number of manipulations of or maneuvers on the catheter in order to adjust the loop size while it is inserted into the patient's heart should be limited. Most preferably, the user only needs two 360° turns or less from the distal end to achieve the maximum enlargement.

For defining the direct correlation between the size change and the torque of the first catheter, a number of experiments were conducted using the cryoablation catheter described above. For the test the first catheter was turned about 180° for three times and after each rotation the width and length of the applicator loop was measured. The torque was applied from the proximal end. In a second test setting, the torque was applied from the distal end of the catheter.

As a non-limiting example, a first catheter built of Nylon 12 and with a length of 145 ± 1 cm was used. Five samples of equally manufactured catheters (Catheter 1 to Catheter 5) were used. The results of the tests can be seen in TABLE 1 and TABLE 2 below.

*TABLE 1: Applicator diameter change when the first catheter is turned from the proximal end*

| Proximal Turn | Diameter at Begin | | 180° turn | | 360° turn | | 540° turn | |
|---|---|---|---|---|---|---|---|---|
| | vertical [mm] | horizontal [mm] | vertical [mm] | horizontal [mm] | vertical [mm] | horizontal [mm] | vertical [mm] | horizontal [mm] |
| Catheter 1 | 27 | 26 | 31 | 28 | 36 | 30 | 42 | 32 |
| Catheter 2 | 27 | 26 | 30 | 28 | 35 | 29 | 42 | 31 |
| Catheter 3 | 26 | 27 | 29 | 28 | 35 | 30 | 40 | 36 |
| Catheter 4 | 27 | 26 | 30 | 30 | 5 | 34 | 40 | 35 |
| Catheter 5 | 28 | 27 | 30 | 28 | 33 | 30 | 39 | 38 |
| Mean [mm] | 27,0 | 26,4 | 30 | 28,4 | 34,8 | 30,6 | 40,6 | 34,4 |
| Std. Dev. [mm] | 0,63 | 0,49 | 0,63 | 0,80 | 0,98 | 1,74 | 1,20 | 2,58 |

**TABLE 2:** *Applicator diameter change when the first catheter is turned from the distal end*

| Distal Turn | Diameter at Begin | | 180° turn | | 360° turn | | 540° turn | |
|---|---|---|---|---|---|---|---|---|
| | vertical [mm] | horizontal [mm] | vertical [mm] | horizontal [mm] | vertical [mm] | horizontal [mm] | vertical [mm] | horizontal [mm] |
| Catheter 1 | 27 | 26 | 35 | 30 | 39 | 36 | 41 | 36 |
| Catheter 2 | 27 | 26 | 34 | 35 | 40 | 36 | 42 | 38 |
| Catheter 3 | 26 | 27 | 30 | 35 | 40 | 35 | 40 | 38 |
| Catheter 4 | 27 | 26 | 33 | 32 | 39 | 36 | 40 | 38 |
| Catheter 5 | 28 | 27 | 36 | 35 | 39 | 35 | 42 | 37 |
| Mean [mm] | 27,0 | 26,4 | 33,6 | 33,4 | 39,4 | 35,6 | 41,0 | 37,4 |
| Std. Dev. [mm] | 0,63 | 0,49 | 2,06 | 20,60 | 0,49 | 0,49 | 0,89 | 0,80 |

The data in the above tables (TABLE 1 and TABLE 2) show that in both cases it is possible to reach the maximal applicator diameter with only three 180° turns. The observed values display only moderate changes when the second catheter gets curved for insertion into a body or heart chamber.

However, it can be seen that the transmission between position of the turn and loop size differs. That means that the transmission is more direct when the catheter is rotated at the distal position: $27 \pm 0,63$ mm$\rightarrow$ $30 \pm 0,63$ mm$\rightarrow$ $34,8 +.0,98$ mm $\rightarrow 40,6 \pm 1,20$ mm (proximal) and $27 \pm 0,63$ mm4 $33,6 \pm 2,06$ mm4 $39,4 \pm 0,49$ mm $\rightarrow 41 \pm 0,89$ mm (distal) respectively. This different behavior can be explained through the distance between the applicator and the position of rotation force.

Still, all these values are only an example here and can differ if the length of the first catheter, the diameter of the applicator or the material of the first catheter changes.

Correlation between applicator force and rotation

When the size of the applicator increases in the heart, the applicator tube produces more contact pressure, because the vein or the tissue respectively is spanned by the size change. For defining the direct correlation between the force of the applicator tube and the torque of the first catheter, the cryoablation catheter described above was used. For the test, the first catheter was turned about 180° for four times and after each rotation the force was measured. The torque was applied from the distal end or from the proximal end of the catheter.

As non-limiting example a first catheter built of Nylon 12 and with a length of $145 \pm 1$ cm was used. Five samples of equally manufactured catheters (Catheter 1 to Catheter 5) were used. The results of the tests can be seen in TABLE 3 and TABLE 4 below.

**TABLE 3:** *Applicator force when the first catheter is turned from the proximal end*

| Proximal Turn | Force at Begin [N] | 180° turn [N] | 360° turn [N] | 540° turn [N] | 720° turn [N] |
|---|---|---|---|---|---|
| Catheter 1 | 0 | 0,2 | 0,39 | 0,55 | 0,82 |
| Catheter 2 | 0 | 0,11 | 0,27 | 0,45 | 0,71 |
| Catheter 3 | 0 | 0,19 | 0,37 | 0,61 | 0,82 |
| Catheter 4 | 0 | 0,15 | 0,36 | 0,5 | 0,73 |
| Catheter 5 | 0 | 0,14 | 0,33 | 0,52 | 0,71 |
| Mean [N] | 0 | 0,16 | 0,34 | 0,53 | 0,76 |
| Std. Dev. [N] | 0 | 0,04 | 0,05 | 0,06 | 0,06 |

After two 360° rotations (turned from the proximal end), a maximal force of 0,76±0,06 N was reached. The force doubled between one full turn to the other 360° turn. The difference between each group is small (at most 0,06 N).

*TABLE 4: Applicator force when the first catheter is turned from the distal end*

| distal turn | force at begin [N] | 180° turn [N] |
| --- | --- | --- |
| Catheter 1 | 0 | 3 |
| Catheter 2 | 0 | 2,8 |
| Catheter 3 | 0 | 3,2 |
| Catheter 4 | 0 | 2,5 |
| Catheter 5 | 0 | 3,3 |
|  |  |  |
| mean | 0 | 2,96 |
| std | 0 | 0,32 |

Because of the short distance between the distal end of the first catheter and the applicator, the force transmission was more direct from the distal end than from the proximal end. For this reason, it was only possible to execute one 180°turn. Much higher forces could be reached here:2,96 ±0,32 N vs. 0,76±0,06 N (two full rotations from the proximal end), respectively. Again, the difference within the group of five catheters is small after the rotation.
Still all these values are only an example here and can differ if the length of the first catheter, the diameter of the applicator and/or the material of the first catheter changes.

**Claims**

1. An ablation device, comprising

   an ablation applicator (210) for applying ablation energy to an ablation region, the ablation applicator having a first applicator end portion (212), a second applicator end portion (213), and an elongate portion (211) extending between the first (212) and second (213) applicator end portions,
   a first catheter (215) having a first distal end portion and a first proximate end portion (219), the first distal end portion being coupled to the first applicator end portion (212), and
   a second catheter (217) having a second distal end portion and a second proximate end portion, the second distal end portion being coupled to the second applicator end portion (213) to supply an ablation medium to the ablation applicator (210),
   wherein the ablation applicator (210) is adapted to form an applicator shape having an applicator size (D) in a plane perpendicular to a longitudinal axis of the first catheter (215), and
   wherein the coupling between the first distal end portion and the first applicator end portion (212) is adapted to transfer a rotational force applied to the first proximate end portion (219) to the ablation applicator (210) and thereby cause the applicator size (D) to change,
   the ablation device further comprising a handle (220) housing the first proximate end portion (219) and the second proximate end portion, the handle comprising a rotational member for applying the rotational force to the first proximate portion.

2. The ablation device according to the preceding claim, wherein the ablation applicator (210) is pre-shaped to a predetermined applicator shape, in particular to a predetermined applicator shape selected from the group consisting of a loop shape, a helical shape, and an arc shape.

3. The ablation device according to the preceding claim, wherein the ablation applicator (210) comprises a shape memory structure defining the predetermined applicator shape.

4. The ablation device according to the preceding claim, wherein the shape memory structure comprises a shape memory material having a loading plateau value of at least 450 N/mm$^2$ and an unloading plateau value of at least one third of the loading plateau value.

5. The ablation device according to any of the preceding claims, further comprising a rotation limiting mechanism (650) adapted to limit rotation of the first proximate end portion to a predetermined angular range.

6. The ablation device according to any of the preceding claims, further comprising a rotation feedback mechanism adapted to provide a feedback indication to a user when the first proximate end portion is rotated by a predetermined amount.

7. The ablation device according to the preceding claim, wherein the feedback indication is selected from the group consisting of a mechanical indication, a haptic indication, a visual indication, an acoustic indication, and a display indication.

8. The ablation device according to any of the preceding claims, further comprising a rotation blocking mechanism (850) adapted to block rotation of the first proximate end portion when the ablation device is operable in a predetermined state.

9. The ablation device according to any of the preceding claims, further comprising a rotation locking mechanism (1096) adapted to selectively lock the rotational position of the first proximate end portion.

10. The ablation device according to any of the preceding claims, wherein the first catheter comprises an outer layer formed of a biocompatible material having an elastic modulus that is equal to or larger than 1500 MPa.

11. An ablation system comprising

an ablation device according to any of the preceding claims, and
an ablation medium source adapted to supply the ablation medium to the second catheter.


**Patentansprüche**

1. Eine Ablationsvorrichtung, aufweisend

einen Ablationsapplikator (210) zum Aufbringen von Ablationsenergie auf einen Ablationsbereich, wobei der Ablationsapplikator einen ersten Applikator-Endabschnitt (212), einen zweiten Applikator-Endabschnitt (213) und einen länglichen Abschnitt (211) aufweist, der sich zwischen dem ersten (212) und dem zweiten (213) Applikator-Endabschnitt erstreckt,
einen ersten Katheter (215) mit einem ersten distalen Endabschnitt und einem ersten proximalen Endabschnitt (219), wobei der erste distale Endabschnitt mit dem ersten Applikator-Endabschnitt (212) gekoppelt ist, und
einen zweiten Katheter (217) mit einem zweiten distalen Endabschnitt und einem zweiten proximalen Endabschnitt, wobei der zweite distale Endabschnitt mit dem zweiten Applikator-Endabschnitt (213) gekoppelt ist, um ein Ablationsmedium an den Ablationsapplikator (210) zu liefern,
wobei der Ablationsapplikator (210) dazu angepasst ist, eine Applikatorform mit einer Applikatorgröße (D) in einer Ebene senkrecht zu einer Längsachse des ersten Katheters (215) zu bilden, und
wobei die Kopplung zwischen dem ersten distalen Endabschnitt und dem ersten Applikator-Endabschnitt (212) angepasst ist, eine auf den ersten proximalen Endabschnitt (219) ausgeübte Rotationskraft auf den Ablationsapplikator (210) zu übertragen und dadurch eine Änderung der Applikatorgröße (D) zu bewirken,
die Ablationsvorrichtung ferner aufweisend einen Griff (220), der den ersten proximalen Endabschnitt (219) und den zweiten proximalen Endabschnitt aufnimmt, wobei der Griff ein Rotationselement zum Aufbringen der Rotationskraft auf den ersten proximalen Abschnitt aufweist.

2. Die Ablationsvorrichtung nach dem vorhergehenden Anspruch, wobei der Ablationsapplikator (210) zu einer vorbestimmten Applikatorform vorgeformt ist, insbesondere zu einer vorbestimmten Applikatorform, die aus der Gruppe ausgewählt ist, die aus einer Schleifenform, einer spiralförmigen Form und einer Bogenform besteht.

3. Die Ablationsvorrichtung nach dem vorhergehenden Anspruch, wobei der Ablationsapplikator (210) eine Formgedächtnisstruktur aufweist, welche die vorbestimmte Applikatorform definiert.

4. Die Ablationsvorrichtung nach dem vorhergehenden Anspruch, wobei die Formgedächtnisstruktur ein Formgedächtnismaterial mit einem Belastungsplateauwert von mindestens 450 N/mm² und einem Entlastungsplateauwert von mindestens einem Drittel des Belastungsplateauwertes aufweist.

**5.** Die Ablationsvorrichtung nach einem der vorangehenden Ansprüche, ferner aufweisend einen Rotationsbegrenzungsmechanismus (650), der dazu angepasst ist, die Rotation des ersten proximalen Endabschnitts auf einen vorgegebenen Winkelbereich zu begrenzen.

**6.** Die Ablationsvorrichtung nach einem der vorhergehenden Ansprüche, die ferner einen Mechanismus zur Rückmeldung der Rotation aufweist, der dazu angepasst ist, dem Benutzer eine Rückmeldung zu geben, wenn der erste proximale Endabschnitt um einen vorgegebenen Betrag gedreht wird.

**7.** Die Ablationsvorrichtung nach dem vorhergehenden Anspruch, wobei die Rückmeldung aus der Gruppe ausgewählt ist, die aus einer mechanischen Meldung, einer haptischen Meldung, einer visuellen Meldung, einer akustischen Meldung und einer Displaymeldung besteht.

**8.** Die Ablationsvorrichtung nach einem der vorhergehenden Ansprüche, die ferner einen Rotationsblockiermechanismus (850) aufweist, der dazu angepasst ist, die Rotation des ersten proximalen Endabschnitts zu blockieren, wenn die Ablationsvorrichtung in einem vorbestimmten Zustand betreibbar ist.

**9.** Die Ablationsvorrichtung nach einem der vorhergehenden Ansprüche, die ferner einen Rotationsverriegelungsmechanismus (1096) aufweist, der dazu angepasst ist, die Rotationsposition des ersten proximalen Endabschnitts selektiv zu verriegeln.

**10.** Die Ablationsvorrichtung nach einem der vorhergehenden Ansprüche, wobei der erste Katheter eine äußere Schicht aus einem biokompatiblen Material mit einem Elastizitätsmodul von 1500 MPa oder mehr aufweist.

**11.** Ein Ablationssystem aufweisend

eine Ablationsvorrichtung nach einem der vorhergehenden Ansprüche, und
eine Ablationsmediumquelle, die dazu angepasst ist, das Ablationsmedium an den zweiten Katheter zu liefern.


## Revendications

**1.** Dispositif d'ablation, comprenant

un applicateur d'ablation (210) pour appliquer une énergie d'ablation à une région d'ablation, l'applicateur d'ablation présentant une première partie d'extrémité d'applicateur (212), une seconde partie d'extrémité d'applicateur (213), et une partie allongée (211) s'étendant entre les première (212) et seconde (213) parties d'extrémité d'applicateur,
un premier cathéter (215) présentant une première partie d'extrémité distale et une première partie d'extrémité proximale (219), la première partie d'extrémité distale étant couplée à la première partie d'extrémité d'applicateur (212), et
un second cathéter (217) présentant une seconde partie d'extrémité distale et une seconde partie d'extrémité proximale, la seconde partie d'extrémité distale étant couplée à la seconde partie d'extrémité d'applicateur (213) pour fournir un milieu d'ablation à l'applicateur d'ablation (210),
dans lequel l'applicateur d'ablation (210) est adapté pour former une forme d'applicateur présentant une taille d'applicateur (D) dans un plan perpendiculaire à un axe longitudinal du premier cathéter (215), et
dans lequel le couplage entre la première partie d'extrémité distale et la première partie d'extrémité d'applicateur (212) est adapté pour transférer une force de rotation appliquée à la première partie d'extrémité proximale (219) à l'applicateur d'ablation (210) et ainsi amener la taille d'applicateur (D) à changer,
le dispositif d'ablation comprenant en outre une poignée (220) logeant la première partie d'extrémité proximale (219) et la seconde partie d'extrémité proximale, la poignée comprenant un élément de rotation pour appliquer la force de rotation à la première partie proximale.

**2.** Dispositif d'ablation selon la revendication précédente, dans lequel l'applicateur d'ablation (210) est préformé à une forme d'applicateur prédéterminée, en particulier à une forme d'applicateur prédéterminée choisie dans le groupe comprenant une forme de boucle, une forme hélicoïdale et une forme d'arc.

**3.** Dispositif d'ablation selon la revendication précédente, dans lequel l'applicateur d'ablation (210) comprend une structure à mémoire de forme définissant la forme d'applicateur prédéterminée.

**4.** Dispositif d'ablation selon la revendication précédente, dans lequel la structure à mémoire de forme comprend un matériau à mémoire de forme présentant une valeur de plateau de chargement d'au moins 450 N/mm$^2$ et une valeur de plateau de déchargement d'au moins un tiers de la valeur de plateau de chargement.

**5.** Dispositif d'ablation selon l'une quelconque des revendications précédentes, comprenant en outre un mécanisme de limitation de rotation (650) adapté pour limiter la rotation de la première partie d'extrémité proximale à une plage angulaire prédéterminée.

**6.** Dispositif d'ablation selon l'une quelconque des revendications précédentes, comprenant en outre un mécanisme de rétroaction de rotation adapté pour fournir une indication de rétroaction à un utilisateur lorsque la première partie d'extrémité proximale est tournée d'une quantité prédéterminée.

**7.** Dispositif d'ablation selon la revendication précédente, dans lequel l'indication de rétroaction est sélectionnée dans le groupe constitué d'une indication mécanique, d'une indication haptique, d'une indication visuelle, d'une indication acoustique et d'une indication d'affichage.

**8.** Dispositif d'ablation selon l'une quelconque des revendications précédentes, comprenant en outre un mécanisme de blocage de rotation (850) adapté pour bloquer la rotation de la première partie d'extrémité proximale lorsque le dispositif d'ablation peut fonctionner dans un état prédéterminé.

**9.** Dispositif d'ablation selon l'une quelconque des revendications précédentes, comprenant en outre un mécanisme de verrouillage de rotation (1096) adapté pour verrouiller sélectivement la position de rotation de la première partie d'extrémité proximale.

**10.** Dispositif d'ablation selon l'une quelconque des revendications précédentes, dans lequel le premier cathéter comprend une couche extérieure formée d'un matériau biocompatible présentant un module d'élasticité qui est égal ou supérieur à 1 500 MPa.

**11.** Système d'ablation, comprenant

un dispositif d'ablation selon l'une quelconque des revendications précédentes, et
une source de milieu d'ablation adaptée pour fournir le milieu d'ablation au second cathéter.

Fig. 1C

Fig. 1A

Fig. 1B

Fig. 1D

Fig. 2C

Fig. 2A

Fig. 2B

EP 3 883 486 B1

Fig. 3A

Fig. 3B

Fig. 4A

Fig. 4B

Fig. 5A

Fig. 5B

EP 3 883 486 B1

Fig. 6A

Fig. 6B

Fig. 6C

Fig. 6D

EP 3 883 486 B1

Fig. 7A

Fig. 7B

EP 3 883 486 B1

Fig. 8A

Fig. 8B

Fig. 8C

Fig. 8D

EP 3 883 486 B1

Fig. 9A

Fig. 9B

Fig. 9C

EP 3 883 486 B1

Fig. 10B

Fig. 10A

EP 3 883 486 B1

Fig. 11

**EP 3 883 486 B1**

**REFERENCES CITED IN THE DESCRIPTION**

**Patent documents cited in the description**

- US 20050010095 A1
- US 9918772 B
- US 9011425 B
- US 6071274 A
- EP 2252228 A2
- WO 2016196066 A2
- US 20170291008 A
- US 8475450 B
- WO 2013039564 A
- US 7263397 B
- US 2013006194 A
- US 8428691 B

**Non-patent literature cited in the description**

- **PRICKETT et al.** *Cryobiology,* 2010